# EUROPÄISCHE PATENTSCHRIFT

(11) **EP 4 281 030 B1**
(45) Veröffentlichungstag und Bekanntmachung des Hinweises auf die Patenterteilung: **31.12.2025**
(21) Anmeldenummer: 22701890.0
(22) Anmeldetag: 13.01.2022
(51) Int. Cl.: A61K 8/06, A61K 8/19, A61K 8/365, A61K 8/37, A61K 8/39, A61K 8/92, A61Q 19/00

(54) **TEMPERATURSTABILE, MINERALÖLFREIE W/O-EMULSION**
TEMPERATURE-STABLE, MINERAL-OIL-FREE W/O EMULSION
EMULSION E/H SANS HUILE MINERALE STABILISÉE AUX VARIATIONS DE TEMPÉRATURE

(30) Priorität: 25.01.2021 DE 102021200621
(43) Veröffentlichungstag der Anmeldung: 29.11.2023
(73) Patentinhaber: Beiersdorf AG, 22529 Hamburg (DE)
(72) Erfinder: HERWIG, Katharina, 22529 Hamburg (DE); BLECKMANN, Andreas, 22926 Ahrensburg (DE); KOCH, Petra, 22457 Hamburg (DE); ROPETER, Katharina, 22529 Hamburg (DE); BEHRENDT, Theresa, 22459 Hamburg (DE); SCHÜRINGS, Keti, 22119 Hamburg (DE)
(74) Vertreter: Beiersdorf AG
(86) Internationale Anmeldenummer: PCT/EP2022/050583
(87) Internationale Veröffentlichungsnummer: WO 2022/157055

(56) Entgegenhaltungen:
- US-A1- 2015 011 654
- US-A1- 2020 383 896
- DATABASE GNPD [online] MINTEL; 6 April 2018 (2018-04-06), ANONYMOUS: "Carbamid Forte Anti-Callus Foot Cream", XP055916406, retrieved from https://www.gnpd.com/sinatra/recordpage/5579855/ Database accession no. 5579855
- DATABASE GNPD [online] MINTEL; 10 May 2019 (2019-05-10), ANONYMOUS: "Cold Cream", XP055916407, retrieved from https://www.gnpd.com/sinatra/recordpage/6535589/ Database accession no. 6535589

## Beschreibung

Die vorliegende Erfindung betrifft eine kosmetische Wasser-in-Öl-Emulsion (W/O-Emulsion) enthaltend Polyglyceryl-4 Diisostearate/Polyhydroxystearate/Sebacate und hydriertes Rizinusöl (INCI: Hydrogenated Castor Oil), wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe. Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen. In diesen Bereich fällt vor allen Dingen die "dekorative" Kosmetik, die mit Hilfe von auf die Haut aufgetragenen Farbstoffen das Erscheinungsbild der Anwender verändert. Indirekt haben aber auch Reinigungs- und Pflegeprodukte einen positiven Einfluss, da eine saubere, gesunde Haut dem Schönheitsideal der Menschen entspricht.

In jüngerer Zeit gibt es einen verstärkten Trend hin zu "natürlichen" Kosmetika, deren Inhaltsstoffe möglichst nicht mehr aus Erdölprodukten stammen oder chemisch synthetisiert sein sollen. Dieser Trend überlappt sich heutzutage mit dem Trend hin zu "veganen" Produkten. Dabei stellt die Suche nach alternativen Inhaltsstoffen, welche diese Kriterien erfüllen, die Produktentwickler vor besondere Herausforderungen. Denn der Austausch der bekannten Inhaltstoffe wie Mineralöle, Silikonöle, Polyacrylate wird praktisch immer mit Nachteilen im Hinblick auf die Produkteigenschaften erkauft. Die Zubereitungen werden instabil und sensorisch unattraktiv, was sich beispielsweise beim Verteilen der Zubereitung auf der Haut und dem mangelnden Einzugsvermögen unangenehm bemerkbar macht. Diese Probleme treten insbesondere bei Wasser-in-Öl-Emulsionen (W/O-Emulsionen) auf, die sich in jüngerer Zeit wieder einer wachsenden Beliebtheit erfreuen. So neigen W/O-Emulsionen ohne Mineralöle und Mineralwachse sowie ohne Polyethylenglycolethern oder -estern (so genannten PEG-Derivaten) zu einer verstärkten Instabilität. Bei längerer Lagerungsdauer kommt es insbesondere bei höheren Temperaturen schnell zu Phasentrennungen in Form von Öl- und/oder Wasserabscheidungen, die sich auch nicht ohne weiteres durch einen höheren Emulgatorgehalt kompensieren lassen.

Es war daher die Aufgabe der vorliegenden Erfindung, eine W/O-Emulsion ohne Mineralöle und-wachse sowie ohne Polyethylenglycolethern und -estern zu entwickeln, die auch bei höheren Temperaturen lagerstabil ist und keine (oder eine deutlich verringerte) Öl- und Wasserabscheidung aufweist. Darüber hinaus sollte die W/O-Emulsion für verschiedene Ölphasenzusammensetzungen anwendbar sein, also eine hohe Anwendungsbreite hinsichtlich der Formelzusammensetzung gewährleisten und sensorisch ansprechend (nicht fettig, nicht klebrig, leicht in die Haut einziehend) sein.

Überraschend gelöst werden die Aufgaben durch eine kosmetische Wasser-in-Öl-Emulsion (W/O-Emulsion) gemäß einer der Ansprüche 1 bis 4.

Zwar kennt der Fachmann die DE 102017221672, DE 102018217130 und US 2015/011654 A1, doch konnten diese Schriften nicht den Weg zur vorliegenden Erfindung weisen. Darüber hinaus kennt der Fachmann die Datenbank-Einträge in der GNPD-Datenbank Mintel mit den Registrierungsnummern 5579855 (Carbamid Forte Anti-Callus Foot Cream) und 6535589 (Cold Cream), die ebenfalls nicht den Weg zur vorliegenden Erfindung weisen konnten.

Es ist dabei erfindungsgemäß bevorzugt, wenn die Zubereitung hydriertes Rizinusöl (INCI: Hydrogenated Castor Oil) in einer Konzentration von 0,3 bis 1,0 Gewichts-%, bezogen auf das Gesamtgewicht der Zubereitung enthält.

Ferner ist es erfindungsgemäß von Vorteil, wenn die Zubereitung Polyglyceryl-4 Diisostearate/Polyhydroxystearate/Sebacate in einer Konzentration von 0,2 bis 2,0 Gewichts-%, bezogen auf das Gesamtgewicht der Zubereitung enthält, wobei eine Konzentration von 0,5 bis 1,5 Gewichts-%, bezogen auf das Gesamtgewicht der Zubereitung, erfindungsgemäß bevorzugt ist.

Enthält die erfindungsgemäße W/O-Emulsion Diisostearoyl Polyglyceryl-3 Dimer Dilinoleate, so ist es erfindungsgemäß vorteilhaft, wenn die Zubereitung Diisostearoyl Polyglyceryl-3 Dimer Dilinoleate in einer Konzentration von 0,2 bis 2,0 Gewichts-%, bezogen auf das Gesamtgewicht der Zubereitung enthält. Besonders bevorzugt ist eine Konzentration von 0,5 bis 1,5 Gewichts-%.

Darüber hinaus ist es vorteilhaft im Sinne der vorliegenden Erfindung, wenn die Zubereitung ein oder mehrere Öle gewählt aus der Gruppe der Verbindungen Triglyceride enthält. Die erfindungsgemäß vorteilhafte Einsatzkonzentration für diese Triglyceride (Gesamtmenge an Triglyceriden) beträgt dabei von 0,5 bis 5,0 Gewichts-%, besonders bevorzugt 0,75 bis 4,5 Gewichts-%, bezogen auf das Gesamtgewicht der Zubereitung.

Es ist dabei erfindungsgemäß bevorzugt, wenn die Zubereitung Kokoscaprylate/Caprate (INCI: Coco-Caprylate/Caprate) enthält. Die erfindungsgemäß vorteilhafte Einsatzkonzentration für Kokoscaprylate/Caprate (INCI: Coco-Caprylate/Caprate) beträgt dabei von 0,5 bis 5,0 Gewichts-%, bezogen auf das Gesamtgewicht der Zubereitung. Besonders bevorzugt dabei der Konzentrationsbereich von 0,75 bis 4,5 Gewichts%, bezogen auf das Gesamtgewicht der Zubereitung.

Ferner sind erfindungsgemäß vorteilhafte Ausführungsformen der vorliegenden Erfindung dadurch gekennzeichnet, dass die Zubereitung ein oder mehrere Palmitate enthält.

Insbesondere ist es erfindungsgemäß vorteilhaft, wenn die Zubereitung Cetylpalmitat und/oder Isopropylpalmitat enthält.

Enthält die Zubereitung Cetylpalmitat, so wird diese Komponente erfindungsgemäß vorteilhaft in einer Konzentration von 0,2 bis 1% Gewichts-%, bezogen auf das Gesamtgewicht der Zubereitung eingesetzt. Besonders bevorzugte Konzentration ist 0,3 bis 0,8 Gewichts-%

Enthält die Zubereitung Isopropylpalmitat, so wird diese Komponente erfindungsgemäß vorteilhaft in einer Konzentration von 1,0 bis 15,0 Gewichts-%, bezogen auf das Gesamtgewicht der Zubereitung eingesetzt. Besonders bevorzugt 3 bis 12 Gewichts-%.

Enthält die Zubereitung Sheabutter, so wird diese Komponente erfindungsgemäß vorteilhaft in einer Konzentration von 0,5 bis 3,0 Gewichts-%, besonders bevorzugt in einer Konzentration von 0,75 bis 2,5 Gewichts-%, bezogen auf das Gesamtgewicht der Zubereitung eingesetzt.

Enthält die Zubereitung Sonnenblumenwachs (INCI: Helianthus Annuus Seed Cera), so wird diese Komponente erfindungsgemäß vorteilhaft in einer Konzentration von 0,1 bis 0,75 Gewichts-%, besonders bevorzugt in einer Konzentration von 0,1 bis 0,5 Gewichts-%, bezogen auf das Gesamtgewicht der Zubereitung eingesetzt.

Vorteilhafte Ausführungsformen der vorliegenden Erfindung sind ferner dadurch gekennzeichnet, dass die Zubereitung ein oder mehrere Pflanzenöle enthält.

Dabei ist es erfindungsgemäß bevorzugt, wenn die Zubereitung ein oder mehrere Öle gewählt aus der Gruppe der Verbindungen Mandelöl (INCI: Prunus Amygdalus Dulcis Oil) und/oder Sonnenblumenöl (INCI: Helianthus Annuus Seed Oil) enthält.

Enthält die Zubereitung Mandelöl (INCI: Prunus Amygdalus Dulcis Oil), so wird diese Komponente erfindungsgemäß vorteilhaft in einer Konzentration von 0,1 bis 0,5 Gewichts-%, bezogen auf das Gesamtgewicht der Zubereitung eingesetzt.

Enthält die Zubereitung Sonnenblumenöl (INCI: Helianthus Annuus Seed Oil), so wird diese Komponente erfindungsgemäß vorteilhaft in einer Konzentration von 0,1 bis 0,5 Gewichts-%, bezogen auf das Gesamtgewicht der Zubereitung eingesetzt.

Erfindungsgemäß vorteilhafte Ausführungsformen der vorliegenden Erfindung sind darüber hinaus dadurch gekennzeichnet, dass die Zubereitung Magnesiumsulfat enthält. Dabei wird Magnesiumsulfat erfindungsgemäß bevorzugt in einer Konzentration von 0,3 bis 3,0 Gewichts-%, bevorzugt in einer Konzentration von 0,5 bis 2,0 Gewichts-%, bezogen auf das Gesamtgewicht der Zubereitung zugesetzt.

Enthält die Zubereitung Kaliumsorbat, so wird diese Komponente erfindungsgemäß vorteilhaft in einer Konzentration von 0,05 bis 0,5 Gewichts-%, bevorzugt in einer Konzentration von 0,075 bis 0,30 Gewichts-%, bezogen auf das Gesamtgewicht der Zubereitung eingesetzt.

Enthält die Zubereitung Zitronensäure, so wird diese Komponente erfindungsgemäß vorteilhaft in einer Konzentration von 0,05 bis 0,25 Gewichts-%, bezogen auf das Gesamtgewicht der Zubereitung eingesetzt. Diese wird dann durch Zusatz von NaOH teilweise neutralisiert, so dass ein Zitronensäure/Citrat-Puffersystem entsteht.

Erfindungsgemäß bevorzugt wird insbesondere eine Kombination aus Kaliumsorbat und Zitronensäure/Natriumcitrat eingesetzt.

Darüber hinaus kann die erfindungsgemäße W/O-Emulsion weitere Inhaltsstoffe enthalten.

Beisielsweise können weitere pflanzliche Öle aus der Gruppe der Verbindungen Persea Gratissima Oil, Orbignya Oleifera Seed Oil, Argania Spinosa Kernel Oil, Prunus Armeniaca Kernel Oil, Simmondsia Chinensis Seed Oil, Cocos Nucifera Oil, Silybum Marianum Seed Oil, Oenothera Biennis Oil, Olea Europaea Fruit Oil, Vitis Vinifera Seed Oil, Cannabis Sativa Seed Oil, Olus Oil, Vegetable Oil, Gossypium Herbaceum Seed Oil, Arctium Lappa Seed Oil, Macadamia Ternifolia Seed Oil, Macadamia Integrifolia Seed Oil Zea Mays Germ Oil, Prunus Amygdalus Dulcis Oil, Ricinus Communis Seed Oil, Glycine Soja Oil, Helianthus Annuus Hybrid Oil, Sesamum Indicum Seed Oil, Brassica Campestris Seed Oil.

Auch kann die Zubereitung ein oder mehrere Ester der Caprylsäure und/oder Caprinsäure enthalten

In einem solchen Falle ist es erfindungsgemäß bevorzugt, wenn die Ester der Caprylsäure und/oder Caprinsäure gewählt werden aus der Gruppe der Verbindungen Caprylsäure-/Caprinsäuretriglyceride (INCI: Caprylic/Capric Triglyceride), Caprylylcaprylat/Caprat (INCI: CaprylylCaprylate/Caprate), Kokosglyceride (INCI: Coco Glycerides), Ethylhexylcocoat (INCI: Ethyl Hexyl Cocoate), Decylcocoat (INCI: Decyl Cocoate), Isoamylcocoat (INCI: Isoamyl Cocoate), Dicaprylyl Carbonat (INCI: Dicaprylyl Carbonate), Kokoscaprylat (INCI: Coco-Caprylate).

Es ist vorteilhaft im Sinne der vorliegenden Erfindung, wenn die Zubereitung Wachse und/oder Fettalkohole enthält. Dabei werden erfindungsgemäß bevorzugt Wachse bzw. Fettalkohole aus der Liste der folgenden Verbindungen eingesetzt:
Cetearyl Alkohol, Cetyl Alkohol, Stearyl Alkohol, Methyl Palmitate, Myristyl Myristate , Hydrogenated Coco-Glycerides, Myristyl Alkohol , Behenyl Alkohol, Theobroma Cacao Seed Butter, Hydrogenated Rapeseed Oil, Caprylic/Capric/ Myristic/Stearic Triglycerid, Hydrogenated Vegetable Oil, Carnaubawachs, Candelillawachs, Tristearin.

Auch kann die Zubereitung ein oder mehrere lipophile Komponenten gewählt aus der Gruppe der Verbindungen Dicaprylyl Ether, Decyl Oleate, Octyldodecanol, Squalane, Triisostearin, Shea Butter Ethyl Ester, Triheptanoin, Hexyldecylsterat, Isoamyllaurat, enthalten.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche Isoflavonoide, Flavonoide, Kreatin, Kreatinin, Taurin, β-Alanin, Tocopherol, Panthenol, Magnolol, Honokiol, Glycerylglycose, Hyaluronsäure und/oder deren Salze und/oder Licochalcon A enthält.

Insbesondere ist es erfindungsgemäß vorteilhaft, wenn die W/O-Emulsion Glycerin enthält.

### Vergleichsversuch

Es wurden die folgenden Rezepturen hergestellt und ihre Stabilität miteinander verglichen.

| Rezepturbeispiele | Rezeptur 1 | Rezeptur 2 |
|---|---|---|
| INCI | Gew.[%] | Gew.[%] |
| Parfum | 0,5 | 0,5 |
| Prunus Amygdalus Dulcis Oil | 0,4 | 0,4 |
| Glycerin | 5,0 | 5,0 |
| Citric Acid | 0,09 | 0,09 |
| Sodium Citrate + Aqua | 0,17 | 0,17 |
| Potassium Sorbate | 0,20 | 0,20 |
| Tocopherol | 0,06 | 0,06 |
| Aqua | ad 100 | ad 100 |
| Magnesium Sulfate | 1,5 | 1,5 |
| Isopropyl Palmitate | 10 | 10 |
| Vegetable Oil | 1,0 | 1,0 |
| Cetyl Palmitate | 0,3 | 0,3 |
| Butyrospermum Parkii Butter | 1.7 | 1.7 |
| Helianthus Annuus Seed Cera + Ascorbyl Palmitate + Tocopherol + Helianthus Annuus Seed Oil | 0,3 | 0,3 |
| Isopropyl Stearate | 2,5 | 2,5 |
| Hydrogenated Castor Oil | 0,5 | 0 |
| Coco-Caprylate/Caprate | 1,0 | 1,0 |
| Ethylhexyl Cocoate | 2,5 | 2,5 |
| Polyglyceryl-4 Diisostearate/Polyhydroxvstearate/Sebacate | 1,3 | 1,3 |
| Diisostearoyl Polyglyceryl-3 Dimer Dilinoleate | 1,0 | 1,0 |

Direkt nach Herstellung ist kein Unterschied erkennbar.

Die Rezepturen werden bei 40°C gelagert. Bereits nach 30 Tagen Lagerung ist bei Rezeptur 2 eine leichte Ölabscheidung zu erkennen. Diese Instabilität wird nach weiterer Lagerung immer deutlicher. Bei Rezeptur 1 ist keine Instabilität im Laufe der Lagerung zu erkennen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| Rezepturbeispiele | 1 | 2 | 3 | 4 | 5 |
|---|---|---|---|---|---|
| INCI | Gew.[%] | Gew.[%] | Gew.[%] | Gew.[%] | Gew.[%] |
| Parfum | 0,2 | 0,7 | 0.3 | 0.4 | 0,5 |
| Prunus Amygdalus Dulcis Oil | 0,2 | 0,5 | 0,1 | 0,3 | 0,4 |
| Glycerin | 3,0 | 5,0 | 10,0 | 6,0 | 5,0 |
| Citric Acid | 0,09 | 0,09 | 0,09 | 0,09 | 0,09 |
| Sodium Citrate + Aqua | 0,17 | 0,17 | 0,17 | 0,17 | 0,17 |
| Potassium Sorbate | 0,15 | 0,075 | 0,30 | 0,10 | 0,20 |
| Tocopherol | 0,05 | 0,10 | 0,07 | 0,15 | 0,06 |
| Aqua | ad 100 | ad 100 | ad 100 | ad 100 | ad 100 |
| Magnesium Sulfate | 0,5 | 1,5 | 1,0 | 0.8 | 1,5 |
| Isopropyl Palmitate | 0 | 10,0 | 6.0 | 5,0 | 11.0 |
| Vegetable Oil | 3,0 | 2,0 | 2,0 | 3.5 | 1,0 |
| Cetyl Palmitate | 0,6 | 0,5 | 0,4 | 0,8 | 0,3 |
| Butyrospermum Parkii Butter | 1,5 | 1,0 | 2.2 | 0.9 | 1.7 |
| Helianthus Annuus Seed Cera + Ascorbyl Palmitate + Tocopherol + Helianthus Annuus Seed Oil | 0,3 | 0,3 | 0,3 | 0,3 | 0,3 |
| Isopropyl Stearate | 4,0 | 3,0 | 3,5 | 4,5 | 2,5 |
| Hydrogenated Castor Oil | 0,5 | 0,3 | 1,0 | 0,4 | 0,6 |
| Coco-Caprylate/Caprate | 5,0 | 3,0 | 3,5 | 1,0 | 0 |
| Cocos Nucifera Oil | 3 | 0 | 0,5 | 2,5 | 0 |
| Ethylhexyl Cocoate | 3 | 0 | 1 | 0 | 2,5 |
| Polyglyceryl-4 Diisostearate/Polyhydroxvstearate/Sebacate | 0,8 | 1,2 | 1,5 | 1,0 | 1,3 |
| Diisostearoyl Polyglyceryl-3 Dimer Dilinoleate | 1,4 | 0,7 | 0 | 0,8 | 1,0 |
| Caprylic/Capric Triglyceride | 0 | 0 | 0 | 1,0 | 0 |

## Patentansprüche

1. Kosmetische Wasser-in-Öl-Emulsion (W/O-Emulsion) enthaltend
a) Polyglyceryl-4 Diisostearate/Polyhydroxystearate/Sebacate und
b) hydriertes Rizinusöl (INCI: Hydrogenated Castor Oil), wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, **dadurch gekennzeichnet, dass** die Zubereitung Diisostearoyl Polyglyceryl-3 Dimer Dilinoleate enthält.

2. Kosmetische Wasser-in-Öl-Emulsion (W/O-Emulsion) enthaltend
a) Polyglyceryl-4 Diisostearate/Polyhydroxystearate/Sebacate und
b) hydriertes Rizinusöl (INCI: Hydrogenated Castor Oil), wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, **dadurch gekennzeichnet, dass** die Zubereitung Sheabutter und/oder Sonnenblumenwachs (INCI: Helianthus Annuus Seed Cera) enthält.

3. Kosmetische Wasser-in-Öl-Emulsion (W/O-Emulsion) enthaltend
a) Polyglyceryl-4 Diisostearate/Polyhydroxystearate/Sebacate und
b) hydriertes Rizinusöl (INCI: Hydrogenated Castor Oil), wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, **dadurch gekennzeichnet, dass** die Zubereitung Kaliumsorbat enthält.

4. Kosmetische Wasser-in-Öl-Emulsion (W/O-Emulsion) enthaltend
a) Polyglyceryl-4 Diisostearate/Polyhydroxystearate/Sebacate und
b) hydriertes Rizinusöl (INCI: Hydrogenated Castor Oil), wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, **dadurch gekennzeichnet, dass** die Zubereitung eine Mischung aus Zitronensäure und Natriumcitrat enthält.

5. Kosmetische W/O-Emulsion nach Anspruch 1 bis 4, **dadurch gekennzeichnet, dass** die Zubereitung hydriertes Rizinusöl (INCI: Hydrogenated Castor Oil) in einer Konzentration von 0,3 bis 1,0 Gewichts-%, bezogen auf das Gesamtgewicht der Zubereitung enthält.

6. Kosmetische W/O-Emulsion nach einem der vorhergehenden Ansprüche, **dadurch gekennzeichnet, dass** die Zubereitung Polyglyceryl-4 Diisostearate/Polyhydroxystearate/Sebacate in einer Konzentration von 0,2 bis 2,0 Gewichts-%, bezogen auf das Gesamtgewicht der Zubereitung enthält.

7. Kosmetische W/O-Emulsion nach einem der vorhergehenden Ansprüche, **dadurch gekennzeichnet, dass** die Zubereitung Diisostearoyl Polyglyceryl-3 Dimer Dilinoleate in einer Konzentration von 0,2 bis 2,0 Gewichts-%, bezogen auf das Gesamtgewicht der Zubereitung enthält.

8. Kosmetische W/O-Emulsion nach einem der vorhergehenden Ansprüche, **dadurch gekennzeichnet, dass** die Zubereitung ein oder mehrere Öle gewählt aus der Gruppe der Verbindungen Triglyceride enthält.

9. Kosmetische W/O-Emulsion nach einem der vorhergehenden Ansprüche, **dadurch gekennzeichnet, dass** die Zubereitung Kokoscaprylate/Caprate (INCI: Coco-Caprylate/Caprate) enthält.

10. Kosmetische W/O-Emulsion nach einem der vorhergehenden Ansprüche, **dadurch gekennzeichnet, dass** die Zubereitung ein oder mehrere Palmitate enthält.

11. Kosmetische W/O-Emulsion nach einem der vorhergehenden Ansprüche, **dadurch gekennzeichnet, dass** die Zubereitung Cetylpalmitat und/oder Isopropylpalmitat enthält.

12. Kosmetische W/O-Emulsion nach einem der vorhergehenden Ansprüche, **dadurch gekennzeichnet, dass** die Zubereitung ein oder mehrere Pflanzenöle enthält.

13. Kosmetische W/O-Emulsion nach einem der vorhergehenden Ansprüche, **dadurch gekennzeichnet, dass** die Zubereitung ein oder mehrere Öle gewählt aus der Gruppe der Verbindungen Mandelöl (INCI: Prunus Amygdalus Dulcis Oil) und/oder Sonnenblumenöl (INCI: Helianthus Annuus Seed Oil) enthält.

14. Kosmetische W/O-Emulsion nach einem der vorhergehenden Ansprüche, **dadurch gekennzeichnet, dass** die Zubereitung Magnesiumsulfat enthält.

## Claims

1. Cosmetic water-in-oil emulsion (W/O emulsion) comprising
a) polyglyceryl-4 diisostearate/polyhydroxystearate/sebacate and
b) hydrogenated castor oil (INCI: Hydrogenated Castor Oil), wherein the preparation is free from mineral oil, paraffin wax, microcrystalline wax, shellac wax and polyethylene waxes, free from polyacrylates, acrylate/C10-C30 alkyl acrylate crosspolymers and vinylpyrrolidone/hexadecene copolymers, and free from 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), ethylhexyl 2-cyano-3,3-diphenylacrylate (INCI: Octocrylene), parabens (particularly methyl, propyl and butyl paraben), methylisothiazolinone, chloromethylisothiazolinone and DMDM hydantoin, polyethylene glycol ethers or polyethylene glycol esters,
**characterized in that** the preparation comprises diisostearoyl polyglyceryl-3 dimer dilinoleate.

2. Cosmetic water-in-oil emulsion (W/O emulsion) comprising
a) polyglyceryl-4 diisostearate/polyhydroxystearate/sebacate and
b) hydrogenated castor oil (INCI: Hydrogenated Castor Oil), wherein the preparation is free from mineral oil, paraffin wax, microcrystalline wax, shellac wax and polyethylene waxes, free from polyacrylates, acrylate/C10-C30 alkyl acrylate crosspolymers and vinylpyrrolidone/hexadecene copolymers, and free from 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), ethylhexyl 2-cyano-3,3-diphenylacrylate (INCI: Octocrylene), parabens (particularly methyl, propyl and butyl paraben), methylisothiazolinone, chloromethylisothiazolinone and DMDM hydantoin, polyethylene glycol ethers or polyethylene glycol esters,
**characterized in that** the preparation comprises shea butter and/or sunflower wax (INCI: Helianthus Annuus Seed Cera).

3. Cosmetic water-in-oil emulsion (W/O emulsion) comprising
a) polyglyceryl-4 diisostearate/polyhydroxystearate/sebacate and
b) hydrogenated castor oil (INCI: Hydrogenated Castor Oil), wherein the preparation is free from mineral oil, paraffin wax, microcrystalline wax, shellac wax and polyethylene waxes, free from polyacrylates, acrylate/C10-C30 alkyl acrylate crosspolymers and vinylpyrrolidone/hexadecene copolymers, and free from 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), ethylhexyl 2-cyano-3,3-diphenylacrylate (INCI: Octocrylene), parabens (particularly methyl, propyl and butyl paraben), methylisothiazolinone, chloromethylisothiazolinone and DMDM hydantoin, polyethylene glycol ethers or polyethylene glycol esters,
**characterized in that** the preparation comprises potassium sorbate.

4. Cosmetic water-in-oil emulsion (W/O emulsion) comprising
a) polyglyceryl-4 diisostearate/polyhydroxystearate/sebacate and
b) hydrogenated castor oil (INCI: Hydrogenated Castor Oil), wherein the preparation is free from mineral oil, paraffin wax, microcrystalline wax, shellac wax and polyethylene waxes, free from polyacrylates, acrylate/C10-C30 alkyl acrylate crosspolymers and vinylpyrrolidone/hexadecene copolymers, and free from 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), ethylhexyl 2-cyano-3,3-diphenylacrylate (INCI: Octocrylene), parabens (particularly methyl, propyl and butyl paraben), methylisothiazolinone, chloromethylisothiazolinone and DMDM hydantoin, polyethylene glycol ethers or polyethylene glycol esters,
**characterized in that** the preparation comprises a mixture of citric acid and sodium citrate.

5. Cosmetic W/O emulsion according to Claim 1 to 4, **characterized in that** the preparation comprises hydrogenated castor oil (INCI: Hydrogenated Castor Oil) at a concentration of 0.3 to 1.0% by weight, based on the total weight of the preparation.

6. Cosmetic W/O emulsion according to any of the preceding claims, **characterized in that** the preparation comprises polyglyceryl-4 diisostearate/polyhydroxystearate/sebacate at a concentration of 0.2 to 2.0% by weight, based on the total weight of the preparation.

7. Cosmetic W/O emulsion according to any of the preceding claims, **characterized in that** the preparation comprises diisostearoyl polyglyceryl-3 dimer dilinoleate at a concentration of 0.2 to 2.0% by weight, based on the total weight of the preparation.

8. Cosmetic W/O emulsion according to any of the preceding claims, **characterized in that** the preparation comprises one or more oils selected from the group of triglyceride compounds.

9. Cosmetic W/O emulsion according to any of the preceding claims, **characterized in that** the preparation comprises coco-caprylate/caprate (INCI: Coco-Caprylate/Caprate).

10. Cosmetic W/O emulsion according to any of the preceding claims, **characterized in that** the preparation comprises one or more palmitates.

11. Cosmetic W/O emulsion according to any of the preceding claims, **characterized in that** the preparation comprises cetyl palmitate and/or isopropyl palmitate.

12. Cosmetic W/O emulsion according to any of the preceding claims, **characterized in that** the preparation comprises one or more vegetable oils.

13. Cosmetic W/O emulsion according to any of the preceding claims, **characterized in that** the preparation comprises one or more oils selected from the group of almond oil compounds (INCI: Prunus Amygdalus Dulcis Oil) and/or sunflower oil (INCI: Helianthus Annuus Seed Oil).

14. Cosmetic W/O emulsion according to any of the preceding claims, **characterized in that** the preparation comprises magnesium sulfate.

## Revendications

1. Émulsion cosmétique eau-dans-huile (émulsion E/H) contenant
a) du diisostéarate de polyglycéryl-4/polyhydroxystéarate/sébacate et
b) de l'huile de ricin hydrogénée (INCI : Hydrogenated Castor Oil), la préparation étant exempte d'huile minérale, de cire de paraffine, de cire microcristalline, de cire de gomme laque et de cires de polyéthylène, exempte de polyacrylates, de polymères réticulés d'acrylate/acrylate d'alkyle en C10-C30 et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), de 2-cyano-3,3-diphénylacrylate d'éthylhexyle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol, **caractérisée en ce que** la préparation contient du dilinoléate de diisostéaroyl-polyglycéryle-3 dimère.

2. Émulsion cosmétique eau-dans-huile (émulsion E/H) contenant
a) du diisostéarate de polyglycéryl-4/polyhydroxystéarate/sébacate et
b) de l'huile de ricin hydrogénée (INCI : Hydrogenated Castor Oil), la préparation étant exempte d'huile minérale, de cire de paraffine, de cire microcristalline, de cire de gomme laque et de cires de polyéthylène, exempte de polyacrylates, de polymères réticulés d'acrylate/acrylate d'alkyle en C10-C30 et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), de 2-cyano-3,3-diphénylacrylate d'éthylhexyle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol, **caractérisée en ce que** la préparation contient du beurre de karité et/ou de la cire de tournesol (INCI : Helianthus Annuus Seed Cera).

3. Émulsion cosmétique eau-dans-huile (émulsion E/H) contenant
a) du diisostéarate de polyglycéryl-4/polyhydroxystéarate/sébacate et
b) de l'huile de ricin hydrogénée (INCI : Hydrogenated Castor Oil), la préparation étant exempte d'huile minérale, de cire de paraffine, de cire microcristalline, de cire de gomme laque et de cires de polyéthylène, exempte de polyacrylates, de polymères réticulés d'acrylate/acrylate d'alkyle en C10-C30 et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), de 2-cyano-3,3-diphénylacrylate d'éthylhexyle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol, **caractérisée en ce que** la préparation contient du sorbate de potassium.

4. Émulsion cosmétique eau-dans-huile (émulsion E/H) contenant
a) du diisostéarate de polyglycéryl-4/polyhydroxystéarate/sébacate et
b) de l'huile de ricin hydrogénée (INCI : Hydrogenated Castor Oil), la préparation étant exempte d'huile minérale, de cire de paraffine, de cire microcristalline, de cire de gomme laque et de cires de polyéthylène, exempte de polyacrylates, de polymères réticulés d'acrylate/acrylate d'alkyle en C10-C30 et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), de 2-cyano-3,3-diphénylacrylate d'éthylhexyle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol, **caractérisée en ce que** la préparation contient un mélange d'acide citrique et de citrate de sodium.

5. Émulsion cosmétique E/H selon les revendications 1 à 4, **caractérisée en ce que** la préparation contient de l'huile de ricin hydrogénée (INCI : Hydrogenated Castor Oil) en une concentration de 0,3 à 1,0 % en poids par rapport au poids total de la préparation.

6. Émulsion cosmétique E/H selon l'une des revendications précédentes, **caractérisée en ce que** la préparation contient du diisostéarate de polyglycéryl-4/polyhydroxystéarate/sébacate en une concentration de 0,2 à 2,0% en poids par rapport au poids total de la préparation.

7. Émulsion cosmétique E/H selon l'une des revendications précédentes, **caractérisée en ce que** la préparation contient du dilinoléate de diisostéaroyl-polyglycéryle-3 dimère en une concentration de 0,2 à 2,0% en poids par rapport au poids total de la préparation.

8. Émulsion cosmétique E/H selon l'une des revendications précédentes, **caractérisée en ce que** la préparation contient une ou plusieurs huiles choisies dans le groupe des composés triglycérides.

9. Émulsion cosmétique E/H selon l'une des revendications précédentes, **caractérisée en ce que** la préparation contient du caprylate/caprate de coco (INCI : Coco-Caprylate/Caprate).

10. Émulsion cosmétique E/H selon l'une des revendications précédentes, **caractérisée en ce que** la préparation contient un ou plusieurs palmitates.

11. Émulsion cosmétique E/H selon l'une des revendications précédentes, **caractérisée en ce que** la préparation contient du palmitate de cétyle et/ou palmitate d'isopropyle.

12. Émulsion cosmétique E/H selon l'une des revendications précédentes, **caractérisée en ce que** la préparation contient une ou plusieurs huiles végétales.

13. Émulsion cosmétique E/H selon l'une des revendications précédentes, **caractérisée en ce que** la préparation contient une ou plusieurs huiles choisies dans le groupe des composés huile d'amande (INCI : Prunus Amygdalus Dulcis Oil) et/ou huile de tournesol (INCI : Helianthus Annuus Seed Oil).

14. Émulsion cosmétique E/H selon l'une des revendications précédentes, **caractérisée en ce que** la préparation contient du sulfate de magnésium.
